# EUROPEAN PATENT APPLICATION

(11) **EP 4 385 422 A1**
(43) Date of publication of application: **19.06.2024**
(21) Application number: 22212767.2
(22) Date of filing: 12.12.2022
(51) Int. Cl.: A61B 8/08, A61B 8/02, A61B 8/00

(54) **GENERATING ULTRASOUND DATA**

(71) Applicant: Koninklijke Philips N.V., 5656 AG Eindhoven (NL)
(72) Inventor: MATTUR, Shashaank Aswatha, Eindhoven (NL); VAJINEPALLI, Pallavi, Eindhoven (NL); KAUSHAL, Nitesh, 5656AG Eindhoven (NL); RADHAKRISHNAN, Ravindranath, Eindhoven (NL); GEYWITZ, Hansjoerg, Eindhoven (NL); PHAM, Hoa, Eindhoven (NL); GAL, Daniel, Eindhoven (NL)
(74) Representative: Philips Intellectual Property & Standards

(57) **Abstract**

A mechanism for generating ultrasound data of a region of interest of a fetus using an ultrasound system having an ultrasound transducer system. An ROI localization functionality of the ultrasound transducer system is disabled (330) responsive to the region of interest being localized (320) and reactivated (310) responsive to an indication (340) that the position of the region of interest has moved with respect to the ultrasound transducer system. Ultrasound data is produced using information about the localization of the region of interest.

## Description

### FIELD OF THE INVENTION

The present invention relates to the field of ultrasound monitoring.

### BACKGROUND OF THE INVENTION

One of the most common approaches for monitoring a fetus or fetuses is through the use of ultrasound-derived data, or simply ultrasound data. There is an increasing interest in the use of an ultrasound system to produce imageless data responsive to a motion of a region of interest. One example of such imageless data is a fetal heartrate (FHR) signal which represents the motion (heartbeat(s)) of a fetal heart. Accurate identification of fetal heartrate is particularly important for assessing the state of a fetus and to determine whether intervention is required during labor. Another example of imageless data is a diaphragm movement signal which represents motion of a diaphragm of a fetus.

It will be appreciated that the imageless data may, in practice, be presented in a graphical form. Thus, the term imageless data refers to data that does not contain a representation of the visual appearance (i.e., image) of any anatomical feature or element.

In traditional ultrasound systems for producing imageless data, a clinical expert will place an ultrasound transducer system on the abdomen of a pregnant individual, and then manipulate the transducer system until it is positioned over the region of interest (e.g., the fetal heart). The clinical expert will then adjust a depth setting on the ultrasound transducer system until the transducer system is correctly monitoring the region of interest at the right depth, i.e., producing imageless data responsive to a motion of the region of interest.

Traditional ultrasound systems have a number of drawbacks. In particular, optimal placement of the transducer system is a challenge, and the expertise of an operating personnel is not always sufficient to match this. It is also recognized that incorrect placement or configuration of the ultrasound transducer system (e.g., to set the correct depth) can cause incorrect or low quality capture of the imageless data, such as imageless data that is highly sensitive to other motions (e.g., a motion of the pregnant individual or a motion of the pregnant individual's heart).

There is a therefore a desire to provide an ultrasound system that overcomes these problems of positioning and configuring the ultrasound transducer system. It would be even more advantageous if this goal could be achieved in an energy efficient manner.

### SUMMARY OF THE INVENTION

The invention is defined by the independent claims. The dependent define advantageous embodiments.

According to examples in accordance with an aspect of the invention, there is provided an ultrasound monitoring system for performing ultrasound monitoring of one or more fetuses. The ultrasound monitoring system comprises an input interface and a control system.

The input interface is configured for communicatively coupling with an ultrasound transducer system.

The control system is configured to be operable to perform, when the ultrasound transducer is communicatively coupled to the input interface: a ROI localization functionality, in which the control system controls the ultrasound transducer system to produce first ultrasound data of the one or more fetuses and processes the first ultrasound data to identify the location of a region of interest of the one or more fetuses with respect to the ultrasound transducer system; and a motion monitoring functionality, in which the control system uses the identified location of the region of interest to control the ultrasound transducer system to produce second ultrasound data, representing a smaller region of the one or more fetuses than the first ultrasound data, that changes responsive to a motion of the region of interest.

The control system is also configured to deactivate the ROI localization functionality responsive to the ultrasound transducer system identifying the location of the region of interest; and reactivate the ROI localization functionality responsive to an indicator indicating that the region of interest has moved with respect to the ultrasound transducer system.

The present disclosure provides an approach for facilitating the automated generation of second ultrasound data (representing a smaller region that first ultrasound data) that changes responsive to a motion of a region of interest (e.g., a fetal heartbeat). First ultrasound data (e.g., ultrasound image data) is generated using an ROI localization functionality of an ultrasound transducer system, and is then processed to identify the location of the region of interest (with respect to the transducer system). The identified location is then used to produce the second ultrasound data using a motion monitoring functionality of the ultrasound transducer system. For instance, in the ROI localization functionality, the identified location can be used to identify (for the transducer system) the location that should be monitored by the ultrasound transducer system to produce the second ultrasound data.

One particularly advantageous feature of the proposed approach is to deactivate the ROI localization functionality (of the ultrasound transducer system) when the location of the region of interest is identified. This approach significantly reduces a power consumption of the overall ultrasound system, as the ROI localization functionality can be extremely resource intensive. This approach can also reduce a confusion by a clinician, as they only need to focus upon the second ultrasound data (representing a relatively small region) and are not tempted to review the first ultrasound data (representing a larger region).

Another advantageous feature of the proposed approach is the reactivation of the ROI localization functionality if the location of the region of interest moves (with respect to the ultrasound transducer system). The occurrence of such a movement is indicated by an indicator.

The indicator could be generated separately or apart from the ultrasound monitoring system, e.g., generated responsive to a user input at a user interface (which could be provided by a clinician separately monitoring the fetus(es) and/or ultrasound monitoring system). As another example, the indicator could be generated by a motion sensor coupled to the ultrasound transducer system (as a motion of the transducer system is likely to indicate a change in the relative positions of the transducer system and the region of interest).

Preferably, the first ultrasound data is image data, containing a representation of the anatomical appearance of the region of interest.

In some preferred examples, the second ultrasound data does not contain a representation of the anatomical appearance of the region of interest. Thus, the second ultrasound data is preferably imageless data. By way of example, the second ultrasound data may contain one or more values that changes responsive to a motion of the region of interest but is independent of the visual appearance of the region of interest (e.g., a pulse rate or respiratory rate).

The control system may be configured to process the second ultrasound data to generate the indicator that indicates whether or not the region of interest has moved with respect to the ultrasound transducer system.

This provides a preferred approach for generating the indicator, as the indicator is generated in a self-enclosed system. In particular, it has been identified that certain changes in the second ultrasound data (e.g., loss of quality, unexpected disruptions or values and so on) could be indicative of a change in the location of the region of interest with respect to the ultrasound transducer system.

The control system may be configured to generate the indicator by: processing the second ultrasound data to determine whether or not the second ultrasound data meets at least one of one or more predetermined criteria; responsive to the second ultrasound data meeting at least one of the one or more predetermined criteria, controlling the indicator to indicate that the region of interest has moved with respect to the ultrasound transducer system; and responsive to the second ultrasound data not meeting at least one of the one or more predetermined criteria, controlling the indicator to indicate that the region of interest has not moved with respect to the ultrasound transducer system.

In some examples, the one or more predetermined criteria include at least one of the following: an amplitude of the second ultrasound data falling below a predetermined threshold amplitude; disruption to a periodicity of the second ultrasound data; and/or the presence of one or more anomalous variations in the second ultrasound data.

The one or more predetermined criteria may include the presence of one or more anomalous variations in the second ultrasound data; and the control system may be configured to process the second ultrasound data using a stochastic signal classification algorithm to determine the presence of absence of the one or more anomalous variations in the second ultrasound data.

The ultrasound transducer system may be configured to, when performing the ROI localization functionality, process the first ultrasound data using a machine-learning method to identify the location of the region of interest.

Optionally, the control system is configured to: deactivate the motion monitoring functionality responsive to the ROI localization functionality being activated or reactivated; and activate the motion monitoring functionality responsive to the ultrasound transducer system identifying the location of the region of interest.

The control system is preferably configured to periodically reactive the ROI localization functionality. This ensures that the region of interest is continually monitored and checked, to avoid or reduce a likelihood of erroneous second ultrasound data being produced.

The region of interest may be a single heart of the one or more fetuses and the motion of the region of interest is motion resulting from a heartbeat. There is a particular importance and interest in the monitoring of the fetal heartrate (FHR), as the FHR is a particularly important indicator of the condition of the fetus (e.g., indicating a level of stress or danger to the fetus). It would therefore be particularly advantageous to facilitate monitoring of the fetal heart with a high degree of accuracy.

The first ultrasound data may comprise B-mode ultrasound image data.

The second ultrasound data may comprise Doppler data, and preferably imageless Doppler data. In particular, the second ultrasound data may comprise an ROI motion signal, being a single time-dependent variable that changes responsive to a motion of the region of interest. Examples of ROI motion signals include a fetal heartrate (FHR) signal (representing a heartbeat) or a diaphragm motion signal (representing a motion of the diaphragm).

The control system may be further configured to derive imageless output data from the second ultrasound data. As previously explained, imageless data is data that does not contain a representation of the visual appearance of an element (or information that can be used to derive an image of the visual appearance of an element). For instance, the imageless data may be single time-dependent value or a time-dependent spectrum.

The imageless output data may be provided to a user interface. The user interface may, for instance, be configured to provide a visual representation of the imageless output data, e.g., in the form of a graph or spectrum.

In some examples, the ultrasound transducer system comprises a plurality of ultrasound transducers wherein: the ultrasound transducer system is configured to: when performing the ROI localization functionality, process the first ultrasound data to identify, from the plurality of ultrasound transducers, a first subset of one or more ultrasound transducers that are more proximate to the region of interest than any other ultrasound transducer; and when performing the motion monitoring functionality, produce the second ultrasound data without using any ultrasound transducer not contained in the first subset of one or more ultrasound transducers; and the first subset of one or more ultrasound transducers does not contain all of the plurality of ultrasound transducers.

The ultrasound transducer system may comprise a wearable ultrasound patch carrying a plurality of ultrasound transducers.

There is also proposed a method for controlling an ultrasound monitoring system. The ultrasound monitoring system comprises an input interface for communicatively coupling with an ultrasound transducer system and a control system configured to be operable to perform, when an ultrasound transducer is communicatively coupled to the input interface: a ROI localization functionality, in which the control system controls the ultrasound transducer system to produce first ultrasound data of the one or more fetuses and processes the first ultrasound data to identify the location of a region of interest of the one or more fetuses with respect to the ultrasound transducer system; and a motion monitoring functionality, in which the control system uses the identified location of the region of interest to control the ultrasound transducer system to produce second ultrasound data, representing a smaller region of the one or more fetuses than the first ultrasound data, that changes responsive to a motion of the region of interest,

The computer-implemented method comprises: deactivating the ROI localization functionality responsive to the ultrasound transducer system identifying the location of the region of interest; and reactivating the ROI localization functionality responsive to an indicator indicating that the region of interest has moved with respect to the ultrasound transducer system.

There is also proposed a computer program product comprising computer program code means which, when executed on a computing device having a control system, cause the control system to perform all of the steps of any herein disclosed method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an ultrasound system;
Fig. 2 illustrates an ultrasound transducer system;
Fig. 3 is a flowchart illustrating a method according to an embodiment; and
Fig. 4 is a flowchart illustrating a step for use in a method according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for generating ultrasound data of a region of interest of a fetus using an ultrasound system having an ultrasound transducer system. A ROI (Region Of Interest) localization functionality of the ultrasound transducer system is disabled responsive to the region of interest being localized and reactivated responsive to an indication that the position of the region of interest has moved with respect to the ultrasound transducer system. Ultrasound data is produced using information about the localization of the region of interest.

In this way, the proposed approach provides an automated mechanism for tracking a region of interest and generating ultrasound data for the tracked region of interest. Proposed approaches are based on the realization that once a region of interest has been localized, it is no longer necessary to continue localizing the region of interest whilst generating the ultrasound data with regard to the specific region of interest, thereby saving energy. Examples of such ultrasound data include a fetal heartrate or the like.

Approaches also recognize that the ultrasound data for the specific region of interest can itself be processed to identify a change in the relative location of the region of interest, e.g., without the need to process information about a broader region surrounding the region of interest. This recognition can be exploited to automatically reactivate or enable the ROI localization functionality.

Disclosed embodiments can be employed in any suitable scenario in which fetal monitoring is desired, but find particular use in maternity units of clinical environments such as hospitals or birthing centers.

The present disclosure provides an approach for localizing a region of interest of one or more fetus (e.g., the fetal heart(s)) for which it is desired for ultrasound data about a specific region of interest (e.g., fetal heartrate (FHR) measurements) to be generated. This can be used for helping in positioning for a single or multiple pregnancy using a multi-transducer ultrasound patch. In the case of a multiple pregnancy, it is further possible to automatically localize a region of interest for multiple fetuses so that the relevant ultrasound data is computed and tagged to the correct fetus.

In the context of the present invention, the term "activate" (and its conjugates) is considered synonymous with the term "enable" (and its respective conjugates). Similarly, the term "deactivate" (and its conjugates) is considered synonymous with the term "disable" (and its respective conjugates). Where relevant, these terms could be used interchangeably.

In the context of the present application, the term "image data" is used to refer to data that contains, or can be processed to derive, a representation of a visual appearance of an element. Similarly, the term "imageless data" is used to refer to data that does not contain, and cannot be processed to derive, a representation of a visual appearance of an element. Of course, imageless data can be presented or formatted in a graphical format (e.g., an image of a graph), but would fail to contain a representation of the visual appearance of the element.

If a first element is localized with respect to a second element, then the spatial location/position of the first element is determined with respect to the second element.

A "functionality" may be a mode of operation or capability of a particular element. If a functionality is active/activated or enabled, then that element performs or carries out the functionality. If inactive/deactivated or disabled, then that element does not perform or carry out the functionality.

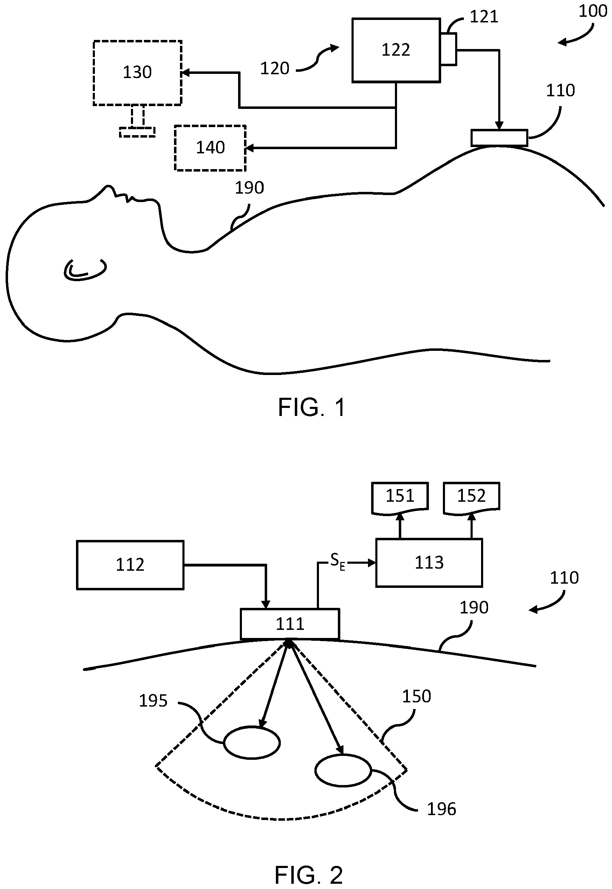

Fig. 1 illustrates an ultrasound system 100, for monitoring one or more fetuses (carried by a subject 190). The ultrasound system 100 comprises an ultrasound transducer system 110 and an ultrasound monitoring system 120 according to a proposed embodiment.

The ultrasound system 100 may comprise a user interface 130 and/or further processing system 140. In other examples, these are absent or form separate elements able to communicate with the ultrasound system 100.

The ultrasound transducer system 110 is configured to produce ultrasound data of an investigated region. Typically, an ultrasound transducer system 110 is configured to transmit ultrasound waves into a subject 190 and receive echo information. The echo information can then be processed by the ultrasound transducer system to generate ultrasound data about the investigated region, i.e., the region into which ultrasound waves were emitted. Examples of ultrasound data include ultrasound image data (e.g., B-mode image data) and imageless data (e.g., data derived from Doppler-based measurement techniques).

Fig. 2 illustrates an example ultrasound transducer system 110. The transducer system comprises one or more transducers 111 (e.g., a plurality of transducers), a beam control system 112 and a processing system 113. Examples of transducers are well known, and include CMUT (Capacitive Micromachined Ultrasonic Transducer) transducers; piezoelectric transducers, formed of materials such as PZT (Lead Zirconate Titanate) or PVDF (Polyvinylidene fluoride); or any other suitable transducer technology.

The one or more transducers 111 are configured to transmit ultrasound waves into the subject 190 responsive to the beam control system 112. Thus, the beam control system 112 controls the transmission of ultrasound waves.

The one or more transducers 111 are also configured to receive echo information (being echoes of the transmitted ultrasound waves from objects 195, 196 in the subject 190) and generate an echo signal S_{E} responsive thereto. The processing system 113 is configured to process the echo signal to produce ultrasound data. Examples of ultrasound data include for example ultrasound image data 151 or (ultrasound) imageless data 152.

The beam control system 112 may also control the generation of the echo signal by the one or more transducers 111.

Of course, the ultrasound transducer system 110 may comprise any necessary circuitry or elements for performing ultrasound monitoring/analysis of a subject. For instance, the beam control system 112 may include beamforming arrangements, beam steering arrangements, drive electronics and/or signal processing circuits (e.g., comprising filtering circuits, sampling circuits, ADCs (Analog to Digital Converters), DACs (Digital to Analog Converters), and so on). Similarly, the processing system 113 may comprise any circuitry necessary for processing an echo signal, e.g., filtering circuits, sampling circuits, ADCs, DACs, and so on.

The detailed structure and operation of a suitable ultrasound transducer system is well known in the art, and is not explained in detail for the sake of conciseness.

The ultrasound transducer system 110 can be controlled to perform at least two different functionalities, including at least an ROI localization functionality and a motion monitoring functionality. In other words, the ultrasound transducer system 110 may be controlled to operate in at least two modes, each mode providing a different functionality.

To perform an ROI localization functionality, the ultrasound transducer system produces and processes first ultrasound data to identify the location of a region of interest with respect to the ultrasound transducer system. The ROI localization functionality therefore identifies the relative location of a region of interest with respect to the ultrasound transducer system.

The first ultrasound data may, for instance, be ultrasound image data that contains a representation of the visual appearance of the investigated region, i.e., the region that receives and reflects ultrasound waves emitted by the one or more transducers 111.

Approaches for producing ultrasound image data are well established, and have been previously described. Approaches for processing ultrasound data to identify the location of a region of interest within the ultrasound data are known, particularly for the processing of ultrasound image data. In particular, the spatial relationship between any given area of image data and the transducer used to produce the image data may be known or determinable.

The ROI is preferably a region that undergoes a periodic motion, such as a fetal heart or a fetal diaphragm. Embodiments are particularly advantageous when the region of interest is a fetal heart, as this is an important structure to monitor during the course of pregnancy and/or birth.

The region of interest may, for instance, be represented by a particular anatomical element or feature (such as the fetal heart or fetal diagram).

To perform a motion monitoring functionality, the ultrasound transducer system produces second ultrasound data that changes responsive to a motion of the region of interest. The second ultrasound data represents a smaller region of the one or more fetuses than the first ultrasound data. In particular, the second ultrasound data should change responsive to changes in the periodicity of the motion of the region of interest.

The second ultrasound data preferably comprises or consists (only) of imageless data. Put another way, in preferred embodiments, the second ultrasound data does not contain a representation of the anatomical appearance of the region of interest.

An example of imageless data is a heartrate measurement, such as a fetal heartrate measurement. Another example is a diaphragm movement measurement, such as a fetal respiratory rate. Approaches for using an ultrasound transducer system to produce imageless data responsive to movement are well known, and include Doppler-based imaging (which employs the principle of Doppler shift to generate imageless data). Typically, such approaches comprise monitoring a position along a particular scanline from a transducer of the ultrasound transducer system to monitor a Doppler shift at said position.

In another example, the second ultrasound data comprises ultrasound image data representing a visual appearance of a smaller region of interest than the first ultrasound data. This approach can allow, for instance, for higher-resolution imaging of a desired region of interest or reduced processing or power requirements to produce an image that still contains the more important region of interest.

In particular, when performing the motion monitoring functionality the ultrasound transducer system is controlled or configured to monitor and target a specific region of interest with respect to the ultrasound transducer system. This can be achieved by setting the depth/distance and the angle of the region of interest from the transducer(s) of the ultrasound transducer system. The ultrasound transducer system may define a scanline that intersects the region of interest and is monitored for changes in intensity, and therefore implied movement, of the region of interest.

The present disclosure provides an ultrasound monitoring system 120 that is configured to control the operation of the ultrasound transducer system. Whilst illustrated as a separate module, for the purposes of illustrative clarity, the ultrasound monitoring system control system 120 may in practice form a part of the ultrasound transducer system (e.g., a processor of the ultrasound transducer system).

The ultrasound monitoring system 120 comprises an input interface 121 for communicatively coupling with the ultrasound transducer system 110. The ultrasound monitoring system also comprises a control system 122 configured to control the operation of the ultrasound transducer system 110 when it is communicatively coupled to the input interface 121. Thus, the control system 122 controls the ultrasound transducer system via the input interface 121.

The control system 122 is configured to control whether or not the ultrasound transducer system performs the ROI localization functionality and whether or not the ultrasound transducer system performs the motion monitoring functionality.

Thus, the control system can be considered to be operable to perform (or not perform) a ROI localization functionality and/or a motion monitoring functionality.

When the control system performs a ROI localization functionality, the control system controls the ultrasound transducer system to produce first ultrasound data of the one or more fetuses and processes the first ultrasound data to identify the location of a region of interest of the one or more fetuses with respect to the ultrasound transducer system.

When the control system performs a motion monitoring functionality, the control system uses the identified location of the region of interest to control the ultrasound transducer system to produce second ultrasound data, representing a smaller region of the one or more fetuses than the first ultrasound data, that changes responsive to a motion of the region of interest.

The control system 122 is configured to deactivate the ROI localization functionality responsive to the ultrasound transducer system identifying the location of the region of interest. Similarly, the control system 120 is configured to reactivate the ROI localization functionality responsive to an indicator indicating that the region of interest has moved with respect to the ultrasound transducer system.

Thus, the control system 122 is configured to control whether or not the ROI localization functionality of the ultrasound transducer system is active.

The principle of the present disclosure is to deactivate the ROI localization functionality once the location of a region of interest has been identified (i.e., once the region of interest has been localized). This provides accurate, but automated, localization of the region for which desired ultrasound data (e.g., imageless data or image data of a smaller region) is to be produced, whilst reducing or avoiding unnecessary energy expenditure by the ultrasound system.

In particular, the production and processing of a large amount of image data is resource intensive. The proposed approach reduces the amount of image data that needs to be produced and processed to still generate or produce useful ultrasound data for a clinician or operator.

If present, the user interface 130 and/or further processing system 140 may be configured to receive at least the second ultrasound data and/or data derived therefrom. For instance, the control system may provide imageless output data to the user interface 130 and/or further processing system. The imageless output data includes data derived from the second ultrasound data. Preferably, the imageless output data does not include data derived from the first ultrasound data.

The user interface 130, if present, may provide a visual representation of the second ultrasound data and/or data derived therefrom (e.g., the imageless output data). Similarly, the further processing system 140, if present, may perform further processing on the second ultrasound data and/or data derived therefrom, e.g., to analyze or generate alerts responsive to the second ultrasound data.

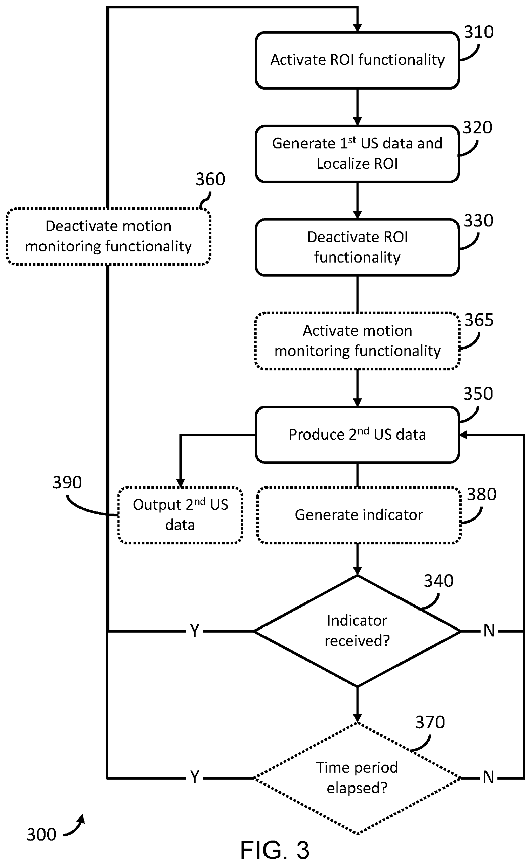

Fig. 3 is a flowchart illustrating the procedure 300 taken by an ultrasound system according to an embodiment.

The procedure 300 comprises a step 310 of activating the ROI localization functionality for the ultrasound transducer system. Step 310 is performed by the ultrasound monitoring system.

In performing the ROI localization functionality, the ultrasound transducer system localizes 320 a region of interest. More particularly, in performing step 320, the ultrasound transducer system produces and processes first ultrasound (1^{st} US) data (of the one or more fetuses) to identify the location of a region of interest of the one or more fetuses with respect to the ultrasound transducer system.

Once the region of interest is localized (i.e., the location of the ROI is identified), the procedure moves to step 330 of deactivating the ROI localization functionality for the ultrasound transducer system. Step 330 is also performed by a control system of the ultrasound monitoring system.

In a determination step 340, it is determined whether or not there is an indicator that indicates that the region of interest has moved with respect to the ultrasound transducer system. Responsive to a positive determination (i.e., there is an indicator), the ROI localization functionality is reactivated, i.e., the method moves to step 310. Responsive to a negative determination, the ROI localization functionality is not reactivated or remains inactive.

Step 340 is performed by the control system.

The procedure 300 also comprises producing 350 second ultrasound (2^{nd} US) data. In particular, in performing a motion monitoring functionality, the control system uses the identified location of the region of interest to control the ultrasound transducer system to produce second ultrasound data that changes responsive to a motion of the region of interest. The second ultrasound data represents a smaller region of the one or more fetuses than the first ultrasound data, e.g., in the form of imageless data.

Although illustrated as a step within the procedure 300, step 350 may in practice be continuously performed. Thus, the motion monitoring functionality may be continually active.

However, in an alternative example, the motion monitoring functionality is deactivated, in a step 360, responsive to the ROI localization functionality being activated or reactivated. Similarly, the motion monitoring functionality may be reactivated, in a step 365, responsive to identifying the location of the region of interest.

This alternative example further improves the power efficiency of the ultrasound system, by avoiding the processing needed to perform the motion monitoring functionality whilst the region of interest is being localized.

In some examples, the method is configured to periodically reactive the ROI localization functionality. Thus, the method 300 may comprise a step 370 of determining whether a predetermined time period has elapsed since the ROI localization functionality was deactivated (in step 330). Responsive to a positive determination (i.e., the predetermined time period has elapsed), the ROI localization functionality is reactivated, i.e., the method moves to step 310. Responsive to a negative determination, the ROI localization functionality is not reactivated or remains inactive.

The predetermined time period may be any suitable value between 1 second and 10 seconds. Preferably, the predetermined time period is no less than 5 seconds.

It will be appreciated that steps 310, 320 and 340, by themselves, represent a computer-implemented method for controlling an ultrasound transducer system of the ultrasound system. These steps are performed by the control system of the ultrasound monitoring system.

More particularly, they represent a method that comprises deactivating the ROI localization functionality responsive to the ultrasound transducer system identifying the location of the region of interest; and reactivating the ROI localization functionality responsive to an indicator indicating that the region of interest has moved with respect to the ultrasound transducer system.

It will be appreciated that the activation or deactivation of any above-described functionality may be overridden, e.g., responsive to a user input. For instance, a user may be able to deactivate any functionality of the ultrasound transducer system or control system to suit their needs/desires using an override input (at a user interface).

It will also be appreciated that any above-described functionality of the ultrasound transducer system may be configured to only be permitted to be active or enabled in certain operation modes of the ultrasound transducer system.

It has previously been explained how, when performing a ROI localization functionality, the ultrasound transducer system is configured to obtain and process first ultrasound data to identify the location of the region of interest.

The first ultrasound data preferably comprises ultrasound image data. Approaches for obtaining ultrasound image data (e.g., B-mode image data) are well-established in the art.

Generally, identifying a region of interest can be performed by processing the ultrasound image data to identify the location of a plurality of bounding boxes, each representing a potential regions of interest, within the image data. Of course, the identified plurality of bounding boxes may undergo non-maximum suppression (or a similar technique) as a filtering technique. A bounding box may, for instance, have a rectangular/cuboidal shape or may otherwise comprise a segmented shape representing the region of interest.

One or more of the bounding boxes may then be selected (e.g., based on a confidence level for each bounding box) as representative of the region of interest. In particular, a bounding box may be selected as a region of interest if a confidence level for that bounding box exceeds some predetermined threshold or the confidence level is within the top B number or percentage of confidence levels, where B is predetermined.

The mapping between any given area of image data and the spatial relationship between the object represented by that area and the transducer used to produce the image data may be known in advance or determinable. In other words, for any position in ultrasound image data, the distance and direction of the absolute location (in real space) represented by that position from the ultrasound transducer system can be readily determined, e.g., using well established approaches. This facilitates mapping from the selected bounding box to a location in space.

One approach to processing ultrasound image data to identify a region of interest (i.e., during the performance of step 320) is the use of an appropriately trained machine-learning algorithm. An appropriately trained machine-learning algorithm is able to process image data to identify bounding boxes (representing potential regions of interest) within the image data.

One particularly suitable machine-learning algorithm for use in identifying bounding boxes representing potential regions of interest is the You Only Look Once (YOLO) algorithm described in J. Redmon and A. Farhadi. Yolov3: An incremental improvement. arXiv preprint arXiv: 1804.02767, 3018. This is a region proposal network which applies a single neural network to a full image to detect certain objects (i.e., regions of interest). More particularly, the neural network divides the image into regions and predicts bounding boxes (i.e., potential regions of interest) and probabilities for each region.

In more detail, the YOLO algorithm divides an input image into an S×S grid. If the centre of an object (i.e., region of interest) falls into a grid cell, then that grid cell is responsible for detecting that object. Each grid cell will determine any bounding boxes (i.e., potential regions of interest) and confidence scores for those boxes. These confidence scores reflect the reliability of the object model contained in the box and the accuracy of the box containing the predicted object.

Each bounding box may be defined by 5 values: x, y, w, h, and confidence. The (x, y) coordinates represent the centre of the box relative to the bounds of the grid cell. The width (w) and height (h) are predicted relative to the whole image. The confidence score (confidence) of the YOLO output is a regression, which is trained to output the intersection of union (IOU) between the output bounding box and ground truth bounding box.

Using the YOLO algorithm it is possible to identify the location of a region of interest within the image data, e.g., the bounding box with the highest confidence or a confidence beyond some predetermined threshold. The center of the bounding box may be used to represent as the specific region of interest, e.g., the location through which a scanline for a motion monitoring functionality is positioned.

Of course, other suitable object or region identification techniques can be used to process image data to identify the location of the region of interest. Examples include any suitable segmentation or object recognition technique, such as those employing a U-net architecture or the like.

Example approaches are disclosed, for instance, by Subramanian, Kalpathi R., Dina M. Lawrence, and M. Taghi Mostafavi. "Interactive segmentation and analysis of fetal ultrasound images." Visualization in Scientific Computing'97. Springer, Vienna, 1997. 115-123; Xu, Lu, et al. "Convolutional-neural-network-based approach for segmentation of apical four-chamber view from fetal echocardiography." IEEE Access 8 (2020): 80437-80446; or Yin, Jintao, et al. "Ultrasonographic Segmentation of Fetal Lung with Deep Learning." Journal of Biosciences and Medicines 9.1 (2021): 146-153. These provide a number of examples for identification of bounding boxes or similar representing regions of interest (e.g., the heart or lungs) within image data.

Once the relative location of the bounding box representing the region of interest within an image has been identified, it is possible to map this location to a spatial location with respect to the ultrasound transducer system. The determined spatial location may, for instance, indicate a distance and angle from the (e.g., center of the) region of interest to the transducer(s) of the ultrasound transducer system. This can facilitate specific monitoring of the region of interest using a motion monitoring technique, e.g., an imageless monitoring technique.

In particular, the mapping between a location within an image and a spatial location is known in advance, for instance, based on known characteristics of the ultrasound transducer system. This is because it is known in which direction ultrasound waves are emitted (and echo information received), as well as a length of time for the echo information to rebound, to thereby facilitate correlation of different areas of the image data to different relative locations in space.

More simply, it is possible to identify a distance and angle that a particular location within an ultrasound image makes with the transducer that produces the ultrasound image.

Previously described approaches for identifying the location of the region of interest assume that the first ultrasound data is ultrasound image data, although approaches for assessing imageless data could be used by the skilled person.

For instance, the first ultrasound data may comprise imageless data representing, for each of a plurality of positions in space, an imageless value responsive to a motion at said position. Each imageless value may be analyzed to identify any positions exhibiting periodic motion, which may indicate the position of a potential region of interest (e.g., a fetal heart or fetal diaphragm). The position having the largest peak-to-peak amplitude change may be identified as the location of the region of interest. This provides an exemplary technique for identifying the location of the region of interest without the need for image data.

However, the use of image data for the first ultrasound data is preferred, as this provides more accurate identification of a desired region of interest, as the appearance of an element that is not a region of interest is less likely to be incorrectly attributed to the region of interest compared to a motion (e.g., which can be caused by external factors such as a material heart or the like).

It has been previously described how the ROI location functionality is reactivated responsive to the indicator indicating that the region of interest has moved with respect to the ultrasound transducer system.

In one example, the indicator may be configured to indicate that the region of interest has moved responsive to a user input (e.g., provided at a user interface). This allows an operator of the ultrasound system to control the reactivation of the ROI location functionality, e.g., if they determine or believe that the region of interest has moved.

In another example, the indicator may be generated by processing the second ultrasound data. Thus, the method 300 of Fig. 3 may comprise a step 380 of processing the second ultrasound data to generate the indicator that indicates whether or not the region of interest has moved with respect to the ultrasound transducer system.

Processing the second ultrasound data may comprise processing the second ultrasound data to determine whether or not the second ultrasound data meets at least one of one or more predetermined criteria. The one or more criteria may be criteria that indicate whether or not the region of interest has moved. Thus, each criterion should be a criterion that is at least partially responsive to a change in location of the region of interest with respect to the ultrasound transducer system.

Accordingly, responsive to the second ultrasound data meeting at least one of the one or more predetermined criteria, step 380 may comprise controlling the indicator to indicate that the region of interest has moved with respect to the ultrasound transducer system; and responsive to the second ultrasound data not meeting at least one of the one or more predetermined criteria, step 380 may comprise controlling the indicator to indicate that the region of interest has not moved with respect to the ultrasound transducer system.

Various criteria can indicate or respond to a change in position of the region of interest. A non-exhaustive number of examples are hereafter provided.

As one example, a first criterion may be an amplitude (e.g., an average or peak amplitude) of the second ultrasound data falling below a predetermined threshold amplitude. If the value(s) of the second ultrasound data diminishes significantly, this is a sign of an anomalous reading and therefore a sign that the region of interest has moved with respect to the ultrasound transducer system. In particular, it may be due to casualty or a change in the localized position of the region of interest due to maternal or fetal movement, either of which would call for intervention.

As another example, a second criterion may be a disruption to a periodicity in the second ultrasound data. If a periodicity of the second ultrasound data undergoes a sudden shift or change, then this is indicative that the region of interest has moved or been repositioned.

By way of example only, the second criterion may be that there is a change of more than X in the periodicity of the second ultrasound data within a time window of Y seconds, where X and Y are predetermined. As a working example, if the region of interest is a fetal heart and the second ultrasound data is a value representing a fetal heartrate, the value of X may be between 10 and 30bpm (e.g., 20bpm) and the value of Y may be between 1 and 3 seconds (e.g., 2 seconds).

As yet another example, a third criterion may be the presence of one or more anomalous variations in the second ultrasound data. Anomalous variations may indicate the presence of another pulsating or moving component (i.e., other than the region of interest) influencing the second ultrasound data, indicating that the region of interest has moved.

To determine whether the third criterion has been met, the second ultrasound data may be processed using an appropriate technique, such as a stochastic signal analysis technique, to identify the presence or absence of any anomalous variations in the second ultrasound data. A suitable examples of a stochastic signal analysis technique is disclosed by Bechet, P., R. Mitran, and M. Munteanu. "A noncontact method based on multiple signal classification algorithm to reduce the measurement time for accurately heart rate detection." Review of Scientific Instruments 84.8 (2013): 084707.

The above-identified example criteria are particularly advantageous when the second ultrasound data is imageless data.

As previously explained, the generated indicator may indicate whether or not one or more of these criteria have been met (i.e., whether or not the region of interest has moved with respect to the ultrasound transducer system). The generated indicator may indicate that the region of interest has moved if any of these criteria are met. Of course, it is not essential that all of the above mentioned criteria be monitored in the process 380, rather only a subset (i.e., not all) may be monitored.

Referring back to Fig. 1, in some examples, the ultrasound transducer system 110 comprises a plurality of transducers. For instance, the ultrasound transducer system may comprise an array of MxO transducers, e.g., in the form of a wearable ultrasound patch carrying a plurality of ultrasound transducers. The value of MxO may be represented by N.

In such examples, when performing the ROI functionality, the first ultrasound data produced by the plurality of transducers may be processed to identify a first subset (of one or more ultrasound transducers) that are more proximate to the region of interest than any other ultrasound transducer.

The identified first subset (and no other transducer) can then be used to produce the second ultrasound data in the motion monitoring functionality. This approach further improves the efficiency of the ultrasound system, by avoiding the need to operate unnecessary or redundant transducers (as the most proximate transducer(s) will produce the most sensitive ultrasound data to motion of the region of interest.

The first subset of one or more ultrasound transducers may comprise a single ultrasound transducer.

The method 300 may further comprise a step 390 of outputting the second ultrasound data. In particular, step 390 may comprise controlling a user interface (not shown), to provide a visual representation of the second ultrasound data, e.g., in graphical form.

More particularly, step 390 may comprise providing imageless output data to a user interface. The imageless output data includes data derived from the second ultrasound data. Preferably, the imageless output data does not include data derived from the first ultrasound data.

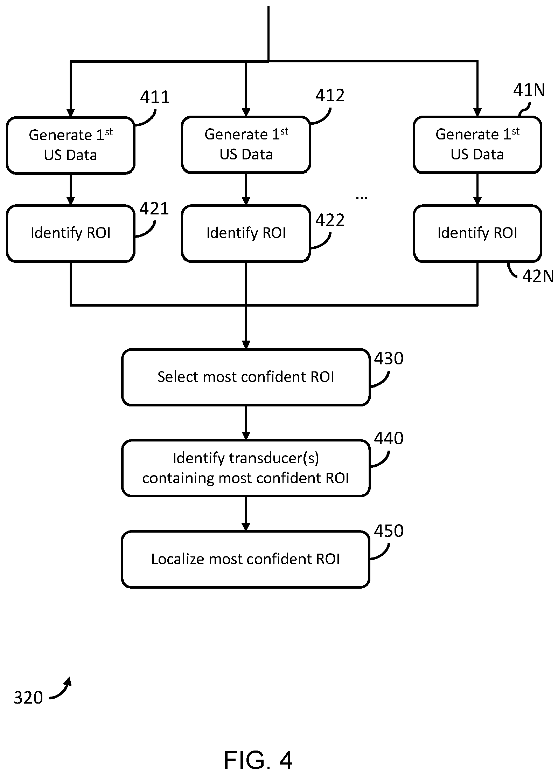

Fig. 4 illustrates one approach for performing step 320, i.e., the ROI functionality, which includes a process for identifying a first subset of one or more transducers of a plurality of N transducers.

In this scenario, each transducer of the ultrasound transducer system is configured to generate an instance of first ultrasound data (e.g., image data). Thus, each of N transducers generates 411, 412, 41N an instance of first ultrasound data. Each instance of first ultrasound data is then processed 421, 422, 42N to identify any potential regions of interest in each instance of first ultrasound data. Where the first ultrasound image data comprises image data, this procedure can be performed using any suitable object detection algorithm, such as a machine-learning method (e.g., the YOLO algorithm previously described).

The method 320 then performs a step 430 of selecting the most confident potential region of interest as the region of interest. This thereby identifies the potential region of interest that best represents a desired region within all of the first ultrasound data produced by the plurality of transducers.

The transducer(s) containing the most confident potential region of interest is then identified in a step 440, thereby identifying the first subset of one or more ultrasound transducers.

The region of interest is then localized, e.g., using the first ultrasound data produced for the identified transducer(s). As previously explained, for image data, the spatial relationship between any given area of image data and the transducer used to produce the image data may be known or determinable.

An alternative to processing first ultrasound data produced by each/all of the N transducers is to individually enable each transducer in turn (i.e., one by one). Each time a transducer is enabled, the first ultrasound data produced is processed in an attempt to identify a region of interest (e.g., for image data, a bounding box having a confidence level beyond some predetermined threshold). If no region of interest is identified, then the current transducer is disabled and the next transducer enabled. This process can be repeated until the region of interest is identified. This provides a more energy efficient approach for identifying the region of interest.

The use of an ultrasound transducer system comprising a plurality of transducers provides a large field of view for the ultrasound system. This provides more efficient identification of the region of interest, e.g., compared to an approach that requires hunting for the region of interest using a smaller handheld ultrasound probe.

Each of the plurality of transducers may share the same drive electronics. The multiple transducers may be mounted on a flexible interconnect and multiplexed to the drive electronics by making use of a bias electrode of each device. The bias voltage controls every single transducer to transmit and receive ultrasound.

In previously described embodiments, it is generally assumed that there is only a single region of interest (e.g., identifying a single heart of a single fetus).

However, it would be advantageous if the ultrasound system and/or method could be adapted to identify (and generate ultrasound data for) a plurality of regions of interest, e.g., multiple fetal hearts for a multiple pregnancy.

In principle, the described approaches for identifying a region of interest can be readily modified to identify a plurality of regions of interest.

For instance, if the first ultrasound data comprises image data, then if multiple bounding boxes (representing multiple potential regions of interest) are identified, then all regions of interest having a confidence level beyond some predetermined threshold or the X most confident bounding boxes can be selected. The value for X can be provided via a user input at a user interface, e.g., if a total number of expected regions of interest are known.

In another scenario, the first ultrasound data comprises imageless data that represents, for a plurality of locations, a value that changes responsive to a motion at that location. Locations having values that change periodically may be identified, and the values having the X largest peak-to-peak amplitude change may be identified. The value for X can be provided via a user input at a user interface, e.g., if a total number of expected regions of interest are known.

It is noted that, when multiple fetuses are present, a problem with existing monitors is the inability to differentiate between multiple regions of interest (e.g., hearts) for the different fetuses, i.e., which ultrasound data (for a specific region of interest) corresponds to which fetus.

This problem can be resolved by combining information from a ROI localization technique (such as those previously described), followed by a real time pose estimator and information from one or more IMU (inertial measurement unit) sensors. In particular, each transducer of the ultrasound transducer system may be associated with a respective IMU sensor.

Thus, to track multiple regions of interest in the case of a multiple pregnancy, it is possible to use a pose identification method together with the information provided by the orientation sensors. In this way, it is possible to correctly tag the right region of interest with the corresponding ultrasound data, which is absent in current ultrasound monitoring systems. More particularly, in current methods, it is not possible to differentiate which ultrasound data represents which fetus in the case of multiple pregnancy.

In a proposed approach, the ultrasound transducer system may be configured to estimate a pose for each identified region of interest (e.g., each fetal heart). Tracking a pose may be functionally equivalent to tracking the location of two or more points of the bounding box representing a region of interest, e.g., no fewer than 8 points, e.g., no fewer than 12 points. This identification can be performed using a machine-learning algorithm such as a neural network (e.g., a deep neural network). The relative orientation of the two or more points with respect to one another gives the orientation of the region of interest. An axis can define the absolute orientation of the region of interest.

Sensor information can be fused together with the pose information extracted from each identified region of interest. In particular, each IMU sensor gives us the position and orientation of the fan beam geometry of a respective transducer (which produced the image data containing the corresponding bounding box) within a world co-ordinate system. The orientation and position of each region of interest can thereby be mapped to a world coordinate system.

The position and orientation of each region of interest can then be tracked based on a nearest neighbour linear approximation from the previous position and orientation. Subsequent acquisition (e.g., if the ROI functionality is reactivated) can be performed in a limited field of view knowing that the lie of the fetus is a gradual movement (unlike limb movement). If at any point, the region of interest is not detected in the search window, the FOV (Field Of View) is broadened in line with the ALARA (As Low As Reasonably Achievable) principle.

In this way, the movement of the region of interest can be tracked.

It will be appreciated that, in any above described embodiments, any generated instance of ultrasound data may be provided to a further processing system and/or output to a user interface for display. In particular, the second ultrasound data and/or data derived therefrom may be provided for display at a user interface.

For instance, the control system may be configured to provide imageless output data to a user interface or further processing system wherein the imageless output data includes data derived from the second ultrasound data. In particular, the imageless output data may comprise a graph of historic or recorded values for the second ultrasound data, e.g., allowing an individual or processing system to track or monitor fetal heart rate.

The present disclosure may make use of a machine-learning algorithm, e.g., to perform identification of the presence and/or location of any bounding boxes (representing potential regions of interest) within image data.

A machine-learning algorithm is any self-training algorithm that processes input data in order to produce or predict output data. Here, the input data comprises ultrasound image data and the output data comprises a bounding box (in the image data) representing a region of interest such as a fetal heart.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g., the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g., ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example instances of image data. The training output data entries correspond to bounding boxes, within the image data, representing a region of interest such as a fetal heart.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The skilled person would be readily capable of developing a processing system or ultrasound system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessing systems that may be programmed using software (e.g., microcode) to perform the required functions. The processing system may be implemented as a combination of dedicated hardware to perform some functions and one or more programmed microprocessing systems and associated circuitry to perform other functions.

Examples of circuitry that may be employed in various embodiments of the present disclosure include, but are not limited to, conventional microprocessing systems, application specific integrated circuits (ASICs), and field-programmable gate arrays (FPGAs).

In various implementations, the processing system may be associated with one or more storage media such as volatile and non-volatile computer memory such as random access memory (RAM), programmable read-only memory (PROM), erasable PROM (EPROM), and electrically EPROM (EEPROM). The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing system may be implemented by a single processing system or by multiple separate processing units which may together be considered to constitute a "processing system". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An ultrasound monitoring system (120) for performing ultrasound monitoring of one or more fetuses (195, 196), the ultrasound monitoring system comprising:
an input interface (121) for communicatively coupling with an ultrasound transducer system (110); and
a control system configured to be operable to perform, when the ultrasound transducer system is communicatively coupled to the input interface:
a region of interest localization functionality, in which the control system controls the ultrasound transducer system to produce (310) first ultrasound data of the one or more fetuses and processes the first ultrasound data to identify the location of a region of interest of the one or more fetuses with respect to the ultrasound transducer system; and
a motion monitoring functionality, in which the control system uses the identified location of the region of interest to control the ultrasound transducer system to produce (350) second ultrasound data, representing a smaller region of the one or more fetuses than the first ultrasound data, that changes responsive to a motion of the region of interest,
wherein the control system is configured to:
deactivate (330) the region of interest localization functionality responsive to the ultrasound transducer system identifying the location of the region of interest; and
reactivate (310) the region of interest localization functionality responsive to an indicator (340) indicating that the region of interest has moved with respect to the ultrasound transducer system.

2. The ultrasound monitoring system of claim 1, wherein the second ultrasound data does not contain a representation of the anatomical appearance of the region of interest.

3. The ultrasound monitoring system of claim 1 or 2, wherein the control system is configured to process (380) the second ultrasound data to generate the indicator that indicates whether or not the region of interest has moved with respect to the ultrasound transducer system.

4. The ultrasound monitoring system of claim 3, wherein the control system is configured to generate the indicator by:
Processing (380) the second ultrasound data to determine whether or not the second ultrasound data meets at least one of one or more predetermined criteria;
responsive to the second ultrasound data meeting at least one of the one or more predetermined criteria, controlling the indicator to indicate that the region of interest has moved with respect to the ultrasound transducer system; and
responsive to the second ultrasound data not meeting at least one of the one or more predetermined criteria, controlling the indicator to indicate that the region of interest has not moved with respect to the ultrasound transducer system.

5. The ultrasound monitoring system of claim 4, wherein the one or more predetermined criteria include at least one of the following:
an amplitude of the second ultrasound data falling below a predetermined threshold amplitude;
disruption to a periodicity of the second ultrasound data; and/or
the presence of one or more anomalous variations in the data.

6. The ultrasound monitoring system of claim 4 or 5, wherein:
the one or more predetermined criteria includes the presence of one or more anomalous variations in the second ultrasound data; and
the control system is configured to process the second ultrasound data using a stochastic signal classification algorithm to determine the presence of absence of the one or more anomalous variations in the second ultrasound data.

7. The ultrasound monitoring system of any of claims 1 to 6, wherein the control system is configured to, when performing the region of interest localization functionality, process the first ultrasound data using a machine-learning method to identify the location of the region of interest.

8. The ultrasound monitoring system of any of claims 1 to 7, wherein the control system is configured to:
deactivate (360) the motion monitoring functionality responsive to the region of interest localization functionality being activated or reactivated; and
activate (330) the motion monitoring functionality responsive to the ultrasound transducer system identifying the location of the region of interest.

9. The ultrasound monitoring system of any of claims 1 to 8, wherein the control system is configured to periodically reactive (370) the region of interest localization functionality.

10. The ultrasound monitoring system of any of claims 1 to 9, wherein the region of interest is a single heart of the one or more fetuses and the motion of the region of interest is motion resulting from a heartbeat.

11. The ultrasound monitoring system of any of claims 1 to 10, wherein the first ultrasound data comprises B-mode ultrasound data, and the second ultrasound data comprise Doppler-mode data.

12. The ultrasound monitoring system of any of claims 1 to 11, wherein the control system is further configured to provide imageless output data to a user interface wherein the imageless output data includes data derived from the second ultrasound data.

13. The ultrasound monitoring system of any of claims 1 to 12, wherein the ultrasound transducer system comprises a plurality of ultrasound transducers wherein:
the control system is configured to:
when performing the region of interest localization functionality, process the first ultrasound data to identify, from the plurality of ultrasound transducers, a first subset of one or more ultrasound transducers that are more proximate to the region of interest than any other ultrasound transducer; and
when performing the motion monitoring functionality, control the ultrasound transducer system to produce the second ultrasound data using any ultrasound transducer contained in the first subset of one or more ultrasound transducers; and
the first subset of one or more ultrasound transducers does not contain all of the plurality of ultrasound transducers.

14. A computer-implemented method for controlling an ultrasound monitoring system (120) comprising an input interface (121) for communicatively coupling with an ultrasound transducer system (110) and a control system configured to be operable to perform, when an ultrasound transducer is communicatively coupled to the input interface:
a region of interest localization functionality, in which the control system controls the ultrasound transducer system to produce first ultrasound data of the one or more fetuses (195, 196) and processes (320) the first ultrasound data to identify the location of a region of interest of the one or more fetuses with respect to the ultrasound transducer system; and
a motion monitoring functionality, in which the control system uses the identified location of the region of interest to control the ultrasound transducer system to produce (350) second ultrasound data, representing a smaller region of the one or more fetuses than the first ultrasound data, that changes responsive to a motion of the region of interest,
wherein the computer-implemented method comprises:
deactivating (330) the region of interest localization functionality responsive to the ultrasound transducer system identifying the location of the region of interest; and
reactivating (310) the region of interest localization functionality responsive to an indicator indicating that the region of interest has moved with respect to the ultrasound transducer system.

15. A computer program product comprising computer program code means which, when executed on a computing device having a control system, cause the control system to perform all of the steps of the method of claim 14.
